# EUROPEAN PATENT APPLICATION

(11) **EP 4 353 223 A1**
(43) Date of publication of application: **17.04.2024**
(21) Application number: 22804081.2
(22) Date of filing: 20.05.2022
(51) Int. Cl.: A61K 9/127, A61K 31/704, A61K 47/24, A61K 47/28, A61P 35/00

(54) **APPLICATION OF PHARMACEUTICAL COMPOSITION HAVING SPECIFIC DRUG-TO-LIPID RATIO IN ANTITUMOR**

(30) Priority: 21.05.2021 CN 202110558965
(71) Applicant: Highfield Biopharmaceuticals Corporation, HangZhou, Zhejiang 310051 (CN)
(72) Inventor: XU, Yuhong, HangZhou, Zhejiang 310051 (CN); CHEN, Xiaolong, HangZhou, Zhejiang 310051 (CN); JIN, Shanshan, HangZhou, Zhejiang 310051 (CN)
(74) Representative: HGF
(86) International application number: PCT/CN2022/094221
(87) International publication number: WO 2022/242762

(57) **Abstract**

An anti-tumor chemotherapeutic drug liposome having a low drug-to-lipid ratio and an application thereof. The anti-tumor chemotherapeutic drug liposome comprises an anti-tumor drug and a liposome as a carrier, wherein the drug-to-lipid ratio of the anti-tumor drug to the lipid carrier is between 0.01 and 0.15.

## Description

### Technical Field

The present disclosure belongs to the field of medical technology, and particularly relates to the anti-tumor application of a pharmaceutical composition having a specific drug-to-lipid ratio.

### Background Art

The application of liposomal delivery system technology as a drug carrier was first proposed by Alec Bangham in 1961, and the technology has gradually matured. Widely used PEG-modified liposomes (stealth liposomes) have prolonged circulation time and the EPR (passive targeting) effect allows drugs to accumulate in tumor tissues and exert a greater effect. Doxil^{®} doxorubicin-loaded liposome is the first liposomal product to be marketed globally. There are also multiple doxorubicin-loaded liposomal drugs in China, including Duomeisu and Libaosu. In addition, liposomes loaded with various anti-tumor drugs such as paclitaxel-loaded liposome, daunorubicin-loaded liposome, vincristine-loaded liposome, irinotecan-loaded liposome, mitoxantrone-loaded liposome, daunorubicin and cytarabine co-loaded liposome and combinations thereof have been marketed or are about to be marketed.

When liposomes are administered, an important parameter in the formulation of these drug-loaded liposomes is the molar ratio of the drug to the lipid molecule, which may be expressed as mass ratio or molar ratio, and abbreviated as the drug-to-lipid ratio. The larger the drug-to-lipid ratio, the more drug is loaded in each liposome, and the higher the drug loading efficiency. The drug-to-lipid ratio varies due to different molecular weights and physicochemical properties of drugs. The drug-to-lipid ratio of doxorubicin-loaded liposomes in prior art is 0.17, the drug-to-lipid ratio of irinotecan-loaded liposomes in prior art is 0.6, and the drug-to-lipid ratio of other drug-loaded liposomes such as vincristine liposomes is 0.2. For liposomes containing two or more drugs, the effective concentration for drug synergy may be reduced, such as Vyxeos, having a drug-to-lipid ratio of 0.12. It is generally believed that the higher the drug-to-lipid ratio, the higher the drug delivery efficiency, and the better the drug efficacy.

However, through our study, we found that the anti-tumor activity of the carrier liposome decreased instead when the drug-to-lipid ratio exceeded a certain value. Therefore, there is an urgent need in the art to optimize the range of drug-to-lipid ratios of drug-loaded liposomes such that pharmaceutical compositions carried by the liposomes have better anti-tumor activity and lower toxicity, so as to achieve optimal anti-tumor effects.

### Summary of the Invention

The main object of the present disclosure is to provide a new range of drug-to-lipid ratios such that pharmaceutical compositions carried by liposomes within the range have effective anti-tumor activity. In particular, the present disclosure provides a range of drug-to-lipid ratios below that commonly found in the industry, i.e., when the molar ratio is between 0.01 and 0.15, pharmaceutical compositions carried by liposomes having drug-to-lipid ratios within the range have better inhibitory activity on tumor cells than that in prior art.

In the first aspect of the present disclosure, a pharmaceutical composition is provided, comprising a1) an anti-tumor drug; a2) a liposome as a carrier; wherein the molar ratio (drug-to-lipid ratio) of the anti-tumor drug to the lipid carrier is between 0.01 and 0.15.

In another preferred example, the range of the drug-to-lipid ratio is between 0.02 and 0.1, preferably 0.022 - 0.085, for example 0.03, 0.04, 0.05, 0.06, 0.07, 0.08, or 0.09.

In another preferred example, the encapsulation rate of the drug in the pharmaceutical composition is > 90%, preferably > 95%, and more preferably > 98%.

In another preferred example, the particle size of the pharmaceutical composition is between 60 and 180 nm, preferably between 80 and 120 nm, and more preferably 85 nm.

In another preferred example, the PDI of the pharmaceutical composition is between 0.01 and 0.2, more preferably between 0.01 and 0.15.

In another preferred example, the anti-tumor drug is selected from the following group: anthracyclines, platinum-based drugs, fluorouracils, camptothecins, taxanes, immunomodulatory drugs or combinations thereof.

In another preferred example, the anti-tumor drug is an anthracycline, preferably doxorubicin.

In another preferred example, the anti-tumor drug is a camptothecin, preferably irinotecan.

In another preferred example, the anti-tumor drug is a paclitaxel drug, preferably paclitaxel.

In another preferred example, the anti-tumor drug is a platinum-based drug, preferably cisplatin.

In another preferred example, the anti-tumor drug is an immunomodulatory drug, preferably resiquimod (R848).

In another preferred example, when the anti-tumor drug is doxorubicin, the range of the drug-to-lipid ratio is below 0.1, more preferably below 0.9, and more preferably 0.022 - 0.085.

In another preferred example, when the anti-tumor drug is irinotecan, the range of the drug-to-lipid ratio is below 0.2, preferably between 0.1 and 0.2, and more preferably 0.015.

In another preferred example, the lipid carrier is selected from the following group of ingredients consisting of: phosphatidylcholine, phosphatidylglycerol, phosphatidylserine, phosphatidylethanolamine, sphingomyelin, cholesterol, polyethylene glycol glycerol fatty acid ester, and polyethylene glycol glycerol phosphatidylethanolamine.

In another preferred example, the lipid carrier includes phosphatidylcholine and cholesterol.

In another preferred example, the phosphatidylcholine is selected from the following group: hydrogenated soybean phosphatidylcholine (HSPC), soybean phosphatidylcholine (SPC), hydrogenated egg phosphatidylcholine, dilauryl phosphatidylcholine, dimyristoyl phosphatidylcholine, dipalmitoyl phosphatidylcholine, distearoyl phosphatidylcholine, 1-myristoyl-2-palmitoyl-sn-glycero-3-phosphocholine, 1 -palmitoyl-2-stearoyl-sn-glycero-3-phosphocholine, 1 -palmitoyl-2-oleoyl-sn-glycero-3-phosphocholine, 1-stearoyl-2-linoleoyl-sn-glycero-3-phosphocholine, dioleoyl phosphatidylcholine, or combinations thereof.

In another preferred example, the structure of the lipid carrier is a sphere surrounded by a lipid molecule bilayer.

In another preferred example, the lipid carrier is prepared by injection.

In another preferred example, the method for preparing the lipid carrier includes the following steps:
(a) mixing the liposomal raw material and cross-linking agent with ethanol to obtain the lipid raw material alcohol phase solution;
(b) providing an acidic aqueous phase solution having a pH of 3.5 - 6.0 (preferably 4.0 - 5.0);
(c) injecting the lipid raw material alcohol phase solution into the acidic aqueous phase solution, stirring and extruding, and performing dialysis to obtain the lipid carrier.

In another preferred example, the liposomal raw material comprises cholesterol and phosphatidylcholine, wherein the phosphatidylcholine is preferably HSPC and SPC as described above.

In another preferred example, the cross-linking agent is a PEG cross-linking agent, preferably having a molecular weight in the range of 1,000 - 5,000, more preferably, DSPE-MPEG2000.

In another preferred example, the mass ratio of the liposomal raw material to the cross-linking agent is 10 - 30:1, preferably 20:1.

In another preferred example, the molar ratio of the phosphatidylcholine to cholesterol to cross-linking agent is (30- 80):(5 - 40):(0.1 - 10).

In another preferred example, in step (b), the acidic aqueous solution is selected from 1,3-propane disulfonic acid aqueous solution to 1,3-propane disulfonic acid triethylamine aqueous solution.

In another preferred example, the volume ratio of the lipid raw material alcohol phase solution to the acidic aqueous solution is 1:8 - 12.

In another preferred example, in step (c), the injecting includes injecting using a syringe or pumping at a fixed flow rate.

In another preferred example, in step (c), the extruding includes extruding using a polycarbonate filter membrane of a fixed pore size.

In another preferred example, the lipid carrier has a conjugated targeting group thereon.

In another preferred example, the targeting group includes, but is not limited to, a polypeptide, a protein, and an antibody fragment.

In another preferred example, the targeting group is an antibody fragment, wherein the antibody isHER2/neu antibody.

In another preferred example, the targeting group is selected from the following group: Fab' fragment, F(ab')2 fragment, Fab fragment, single chain Fv fragment (scFv), single domain antibody (sdAb), minibody, or combinations thereof.

In the second aspect of the present disclosure, a method is provided for preparing the pharmaceutical composition of the first aspect, including the steps: (1) mixing and incubating an anti-tumor drug solution with a lipid carrier dispersion to obtain the pharmaceutical composition; or (2) adding an anti-tumor drug to a lipid raw material alcohol phase solution or an aqueous phase solution to obtain the pharmaceutical composition in one step during the preparation of a liposome.

In another preferred example, in step (1), the incubation is carried out at 20-65°C, preferably 50 - 60°C.

In another preferred example, in step (1), the incubation time is 10 min - 1 h.

In another preferred example, the method further includes the step: mixing and incubating the obtained drug-loaded liposome with a modified targeting group to obtain the targeted pharmaceutical composition.

In another preferred example, the molar ratio of the liposomal raw material to cross-linking agent to targeting group is (30 - 120):(0.1 - 10):(0.01 - 0.1).

In the third aspect of the present disclosure, a use is provided for the pharmaceutical composition according to the first aspect, for the preparation of b1) an inhibitor that inhibits tumor cell proliferation; b2) an anti-tumor drug.

In another preferred example, the dosage form of the pharmaceutical composition is non-oral.

In another preferred example, the dosage form of the pharmaceutical composition is selected from the following: tablet, capsule, granule, suspension, pill, solution, syrup, or injection.

In another preferred example, the dosage form of the pharmaceutical composition is an injection.

In another preferred example, the injection is selected from the following: liquid formulation, suspension formulation, and lyophilized powder for injection.

In another preferred example, the mode of administration of the injection is selected from the following: intravenous injection, subcutaneous injection, and in situ injection.

In another preferred example, the tumors are selected from the following group: breast cancer, gastric cancer, ovarian cancer, liver cancer, melanoma, colorectal cancer, or combinations thereof.

In the fourth aspect of the present disclosure, a method is provided for inhibiting tumor cell growth in vitro, including the steps: administering to the cell a medically effective amount of the pharmaceutical composition according to the first aspect.

In another preferred example, the cell is a human tumor cell.

In another preferred example, the cell is a chemoresistant human tumor cell.

In another preferred example, the cell is selected from the following: Sk-Br-3, BT-474, NCI-N87, MDA-MB-231, MDA-MB-453, MCF-7, A2780, MGC-803, HUVEC, and MCF-7/Adr.

In the fifth aspect of the present disclosure, a method is provided for preventing and/or treating a tumor, including the steps: administering a medically effective amount of the pharmaceutical composition according to the first aspect to a subject in need thereof.

It should be understood that within the scope of the present disclosure, the various technical features mentioned above and those specifically described in the following sections (such as in the examples) may be combined with each other to form new or preferred technical solutions. Due to space limitations, they will not be enumerated in detail here.

### Description of Attached Drawings

Figure 1 is a cryo-electron microscopy image of doxorubicin-loaded liposomes. (A) A doxorubicin-loaded liposome having a drug-to-lipid ratio of 0.17 (high drug-to-lipid ratio); (B) a doxorubicin-loaded liposome having a drug-to-lipid ratio of 0.043 (low drug-to-lipid ratio).
Figure 2 Pharmacodynamic study of doxorubicin-loaded liposomes having a high drug-to-lipid ratio, a low drug-to-lipid ratio, as well as an antibody-targeted doxorubicin-loaded liposome having a low drug-to-lipid ratio on different cells (A) SK-Br-3, (B) NCI-N87, (C) BT474, (D) MDA-MB-453, (E) MCF-7, (F) A2780, (G) MGC-803, (H) HUVEC, (I) MDA-MB-231.
Figure 3 Inhibitory effect of doxorubicin-loaded liposomes having a high drug-to-lipid ratio, a low drug-to-lipid ratio, as well as an antibody-targeted doxorubicin-loaded liposome having a low drug-to-lipid ratio on MCF-7/Adr Adriamycin-resistant breast cancer cells.
Figure 4 Plasma concentration curve after injection of doxorubicin-loaded liposomes having high, medium, and low drug-to-lipid ratios.
Figure 5 Changes in the drug-to-lipid concentration ratio in blood after injection of doxorubicin-loaded liposomes having high, medium, and low drug-to-lipid ratios.
Figure 6 Effect of doxorubicin-loaded liposomes having a high drug-to-lipid ratio, a low drug-to-lipid ratio, as well as an antibody-targeted doxorubicin-loaded liposome having a low drug-to-lipid ratio on tumor growth inhibition in nude mice BT474 breast cancer xenograft model.
Figure 7 Effect of doxorubicin-loaded liposomes having a high drug-to-lipid ratio, a low drug-to-lipid ratio, as well as an antibody-targeted doxorubicin-loaded liposome having a low drug-to-lipid ratio on body weight changes in mice of the nude mice BT474 breast cancer xenograft model after dosing.
Figure 8 Pharmacodynamic effect of an antibody-targeted doxorubicin-loaded liposome having a low drug-to-lipid ratio (0.043) on HER2/neu-expressing NCI-N87 gastric cancer xenograft model
Figure 9 Pharmacodynamic effect of antibody-targeted irinotecan-loaded liposomes having different drug-to-lipid ratios on NCI-N87 tumor cells.
Figure 10 In vivo pharmacodynamic effect of a R848-loaded liposome having a low drug-to-lipid ratio on hHER2-MC38 colorectal cancer model.

### Specific Examples

After extensive and in-depth research, the inventors of the present disclosure unexpectedly found that when the drug-to-lipid ratio of the anti-tumor drug and lipid carrier composition was between 0.01 and 0.15, although the drug loading efficiency at the unit lipid concentration was reduced, the anti-tumor effect at the unit drug concentration was better and had greater in vivo and in vitro anti-tumor effects. On this basis, the present disclosure was completed.

In particular, the present disclosure provides examples in which doxorubicin-loaded liposomes, irinotecan-loaded liposomes, paclitaxel-loaded liposomes, resiquimod-loaded liposomes, and cisplatin-loaded liposomes are prepared and studied. Examples of the present disclosure showed that when the drug-to-lipid ratio of the doxorubicin-loaded liposome was within the range of 0.02 - 0.08 (the drug-to-lipid ratio in prior art is 0.17), the efficacy of the drug-loaded liposomes was greatly improved, and in vivo pharmacokinetic data showed that the circulating half-life of liposomes having a low drug-to-lipid ratio was prolonged and the stability was improved. The in vivo anti-tumor effect was also improved. Both irinotecan-loaded liposomes having a drug-to-lipid ratio of 0.075 and 0.015 were superior to liposomes with a drug-to-lipid ratio of 0.3. In particular, when the surface of the liposome is connected to the polypeptide/protein/antibody molecule that targets tumor-associated antigen (TAA), active-targeting liposomes having a low drug-to-lipid ratio have excellent effects on tumor cells with high, medium and low TAA expression, and great inhibitory effects on chemoresistant tumor cells.

### Terms

As used herein, "pharmaceutical composition of the present disclosure" refers to a pharmaceutical composition comprising an anti-tumor drug and a liposome, and the composition has a drug-to-lipid ratio within the range of 0.01 - 0.15, preferably 0.02 - 0.1.

### A Pharmaceutical Composition and a Method of Administration

The present disclosure provides a pharmaceutical composition comprising a1) anti-tumor drug; a2) a liposome as carrier; wherein the molar ratio (drug-to-lipid ratio) of the anti-tumor drug to the liposome carrier is between 0.01 and 0.15.

The anti-tumor drug in the pharmaceutical composition of the present disclosure is selected from the following group: anthracyclines, platinum-based drugs, fluorouracils, camptothecins, taxanes, immunomodulatory drugs or combinations thereof.

The lipid molecules in the pharmaceutical composition described herein are phospholipids and related amphiphilic molecules commonly used in the industry, selected from phosphatidylcholine, phosphatidylglycerol, phosphatidylserine, phosphatidylethanolamine, sphingomyelin, cholesterol, polyethylene glycol glycerol fatty acid ester, and polyethylene glycol glycerol phosphatidylethanolamine.

In another preferred example, the composition of the liposome is a sphere surrounded by a bilayer self-assembled from lipid molecules.

In another preferred example, the liposome has a conjugated targeting group thereon.

In another preferred example, the targeting group includes, but is not limited to, a polypeptide, a protein, and an antibody fragment.

In another preferred example, the targeting group is selected from the following group: Fab' fragment, F(ab')2 fragment, Fab fragment, single chain Fv fragment (scFv), single domain antibody (sdAb), minibody, or combinations thereof.

As used herein, the term "effective amount" or "effective dose" refers to an amount that is capable of producing a function or activity (i.e., an anti-tumor activity) on a human and/or animal and is acceptable by the human and/or animal.

As used herein, the term "pharmaceutically acceptable" ingredient is a substance that is suitable for use in humans and/or mammals without excessive adverse side effects (e.g., toxicity, irritation, and allergic reactions), i.e., a substance that has a reasonable benefit/risk ratio. The term "pharmaceutically acceptable carrier" refers to a carrier for the administration of a therapeutic agent, including various excipients and diluents.

The pharmaceutical composition of the present disclosure contains a safe and effective amount of the active ingredient of the present disclosure and a pharmaceutically acceptable carrier. Such carriers include (but are not limited to): saline, buffer, glucose, water and combinations thereof. Generally, the pharmaceutical formulation should match the mode of administration. The dosage form of the pharmaceutical composition of the present disclosure is selected from the following: tablet, capsule, granule, suspension, pill, solution, syrup, or injection.

In another preferred example, the dosage form of the pharmaceutical composition is an injection.

In another preferred example, the injection is selected from the following: injection, suspension, lyophilized powder for injection.

In another preferred example, the mode of administration of the injection is selected from the following: intravenous injection, subcutaneous injection, and in situ injection.

Preparation is carried out by conventional methods with saline or an aqueous solution containing glucose and other adjuvants. The pharmaceutical composition is preferably manufactured under sterile conditions.

The effective amount of the active ingredient of the present disclosure may vary with the mode of administration and the severity of the disease to be treated. Selection of a preferred effective amount may be determined (e.g., by clinical trials) by those of ordinary skill in the art based on various factors. Such factors include, but are not limited to the pharmacokinetic parameters of the active ingredient, such as bioavailability, metabolism, half-life, etc.; the severity of the patient's disease to be treated, the patient's weight, the patient's immune status, the route of administration, etc. Generally, when the active ingredient of the present disclosure is given at a dose of about 0.00001 mg - 50 mg/kg of animal body weight per day (preferably, 0.0001 mg - 10 mg/kg of animal body weight), a satisfactory effect may be obtained. For example, several separate doses may be given each day, or the dose may be proportionately reduced, as required based on the urgency of the treatment scenario.

Typically, when the pharmaceutical composition of the present disclosure is administered orally, the average daily dose of a subject weighing 60 kg (human) is generally 10 - 500 mg, preferably 20 - 300 mg, and more preferably 50 - 250 mg. The daily dose may be taken in one, two, or more doses.

Pharmaceutically acceptable carriers of the present disclosure include (but are not limited to): water, saline, nanogels, or combinations thereof. The choice of carriers should be compatible with the mode of administration, and this is well known to those of ordinary skill in the art.

### Liposome & Drug-to-lipid Ratio

The application of liposomal delivery system technology as a drug carrier was first proposed by Alec Bangham in 1961. Liposomes are small, spherical vesicles characterized by lipid bilayers, consisting of phospholipids or amphiphilic molecules such as sterols (e.g. cholesterol), with an aqueous phase therein. Drugs are loaded into liposomes or lipid membranes, altering the pharmacokinetics and pharmacodynamics of the drug.

Among the prescriptions of these drug-loaded liposomes, an important parameter is the molar ratio of the drug to the lipid molecule (drug-to-lipid ratio), doxorubicin-loaded liposomes in prior art include Doxil, Duomesu, and Libaosu having a drug-to-lipid ratio of 0.17, Myocet^{®} having a drug-to-lipid ratio of 0.27. In addition, the drug-to-lipid ratio of irinotecan-loaded liposome Onivyde^{™} is 0.6. In addition, the drug-to-lipid ratio of vincristine-loaded liposomes as disclosed in (Cancer Chemother Pharmacol (2013) 71:555 - 564) is 0.2; the total drug-to-lipid ratio of Vyxeos-loaded liposomes disclosed in (International Journal of Pharmaceutics 391 (2010) 248 - 259) is 0.12, and that of cisplatin-loaded liposomes is 0.2.

Vincristine-loaded liposomal formulations having different drug-to-lipid ratios were evaluated in (Biochimica et Biophysica Acta 1758 (2006) 55 - 64), and it was found that the in vivo release half-life T1/2 of vincristine from egg sphingomyelin/cholesterol-loaded liposomes changed with different drug-to-lipid ratios: the half-life was 6.1 h when the drug-to-lipid ratio (D/L) was 0.025 (wt/wt), 15.6 h when the drug-to-lipid ratio (D/L) was 0.1 (wt/wt), and 117 h when the drug-to-lipid ratio (D/L) was 0.6 (wt/wt). This shows that the half-life increases when the drug-to-lipid ratio increases. U.S. Patent US5616341 (High drug:lipid formulations of liposomal antineoplastic agents) disclosed a preparation and application of doxorubicin-loaded liposomes and vincristine-loaded liposomes having a high drug-to-lipid ratio.

**Key Advantages of the Present Disclosure Include the Following:**
(a) The anti-tumor activity of liposomes having a low drug-to-lipid ratio on tumor cells as disclosed in the present disclosure is greater than that of liposomes having a high drug-to-lipid ratio in the prior art.
(b) At the same dose, liposomes having a low drug-to-lipid ratio have a longer residence time in the body and release drugs more slowly.
(c) Liposomes having a low drug-to-lipid ratio have better in vivo anti-tumor effects.

The present disclosure is further described below with specific examples. It should be understood that these examples are provided solely for the purpose of illustrating the present disclosure and are not intended to limit the scope of the present disclosure. In the following examples, experimental methods without specified conditions are typically conducted under conventional conditions or as recommended by the manufacturer. Unless otherwise stated, percentages and weight fractions are weight percentages and parts by weight.

### Example 1 Preparation of an Example of Liposomes Encapsulating Doxorubicin (Adriamycin)

900 mg of HSPC, 300 mg of Chol and 60 mg of DSPE-MPEG2000 were weighed, 2 mL of anhydrous ethanol was added, and heated in a water bath to 60°C. Then, a magnetic stirring bar was added for stirring until complete dissolution, resulting in a clear liquid mixture. At the same time, 3.304 g of ammonium sulfate was placed in a glass bottle, added with 100 mL of ultrapure water, and stirred until dissolution, and the pH was then adjusted to 5.0 to obtain an ammonium sulfate solution with a concentration of 250 mM; this solution was prepared just before use. The dissolved lipid mixture was injected into 19 mL of the ammonium sulfate aqueous solution using a syringe, then stirred and extruded mixture six times, and dialysis was then performed using a 10 kD dialysis membrane in a HEPES buffer (10 mM, pH 6.8) to obtain an empty liposome with an external aqueous phase of a HEPES solution.

In addition, 10 mg/mL of Adriamycin solution was added to the above empty liposome suspension according to the drug-to-lipid ratio set above. The total volume was adjusted to 10 mL, then incubated with stirring at 60°C for 30 min. After drug loading was completed, a 100kD dialysis membrane was used to remove the free drug and detect the content of Adriamycin and HSPC, cholesterol and DSPE-MPEG2000, so as to confirm the molar ratio of drug molecules to total lipid molecules in the Adriamycin-loaded liposome.

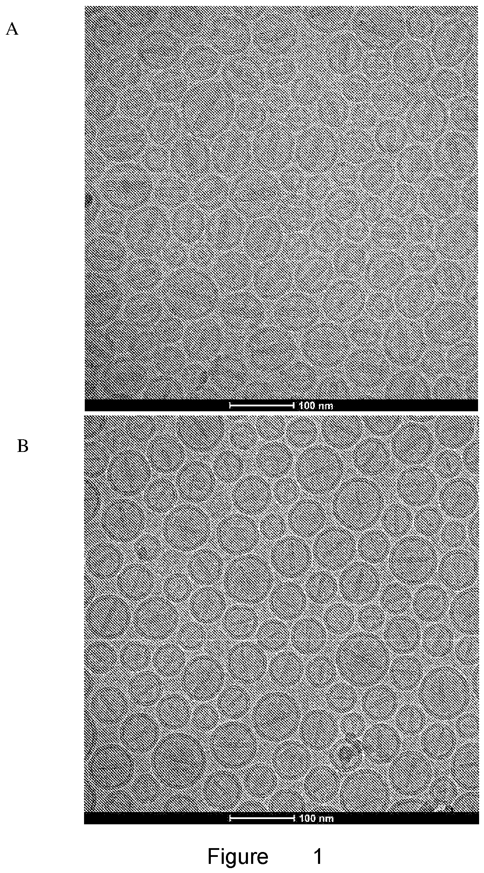

The Nano-S90 nanoparticle size analyzer was used for detection and the particle size of doxorubicin-loaded liposomes having different drug-to-lipid ratios was about 85 nm; the polydispersity index (PDI) was less than 0.1. Among which, the cryo-electron microscopy morphologies of the doxorubicin-loaded liposome formulation having a drug-to-lipid ratio of 0.17 and the doxorubicin-loaded liposome having a drug-to-lipid ratio of 0.043 are as shown in Figure 1A and Figure 1B, and the liposomes are mostly circular in shape, with uniform size and distribution. Among which, doxorubicin crystals in the doxorubicin-loaded liposome having a drug-to-lipid ratio of 0.043 were finer than those in the doxorubicin-loaded liposome having a drug-to-lipid ratio of 0.17.

### Example 2 Efficacy Tested in Doxorubicin-loaded Liposomes Having Different Drug-to-lipid Ratios

In the present example, the cytotoxic effect of doxorubicin-loaded liposomes having different drug-to-lipid ratios that were prepared in Example 1 on tumor cells was assessed.

First, efficacy studies were conducted on tumor cell lines listed in Table 1, including SK-Br-3, NCI-N87, BT474, MDA-MB-453, MCF-7, MGC-803, A2780, MDA-MB-231, and a normal cell line, HUVEC.

**Table 1 Cell Lines**

| Cell Line | Description |
|---|---|
| Sk-Br-3 | Human breast cancer cell line |
| BT-474 | Human breast cancer cell line |
| NCI-N87 | Human gastric cancer cell line |
| MDA-MB-231 | Human triple-negative breast cancer cell line |
| MDA-MB-453 | Human breast cancer cell line |
| MCF-7 | Human breast cancer cell line |
| A2780 | Human ovarian cancer cell line |
| MGC-803 | Human gastric cancer cell line |
| HUVEC | Normal human umbilical vein endothelial cells |
| MCF-7/Adr | Human breast cancer doxorubicin-resistant cell line |

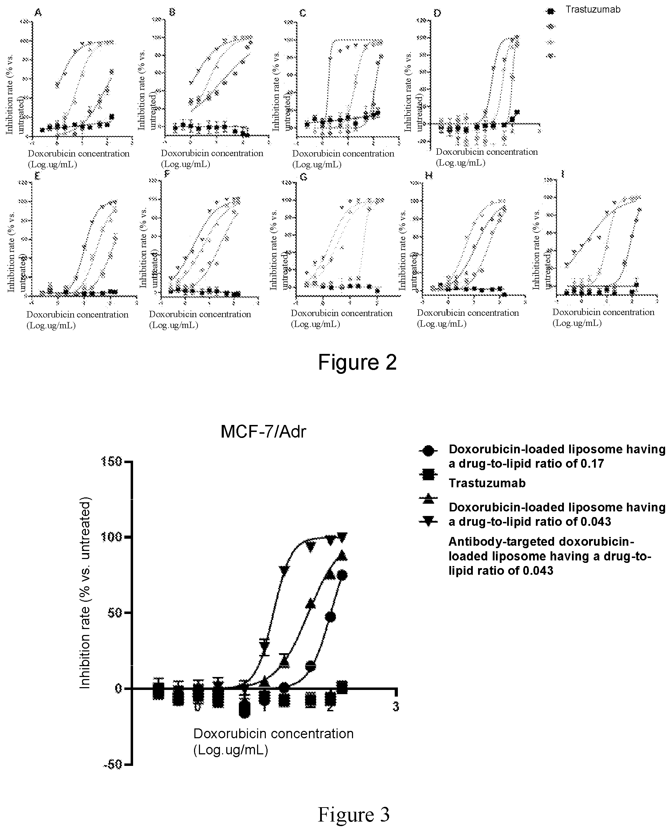

The cytotoxic effect of doxorubicin-loaded liposomes having different drug-to-lipid ratios on tumor cells was assessed using the CellTiter-Glo^{®} Luminescent Cell Viability Assay kit from Promega Corporation. Graph Prism 8.0 analysis software was used to fit cell cytotoxicity curves and calculate the respective IC50 values. See Figure 2 and Table 2 for the results.

The experimental results showed that doxorubicin-loaded liposomes having drug-to-lipid ratios of 0.022, 0.043, and 0.085 had greater cell cytotoxic activity than doxorubicin-loaded liposomes having a high drug-to-lipid ratio (0.17).

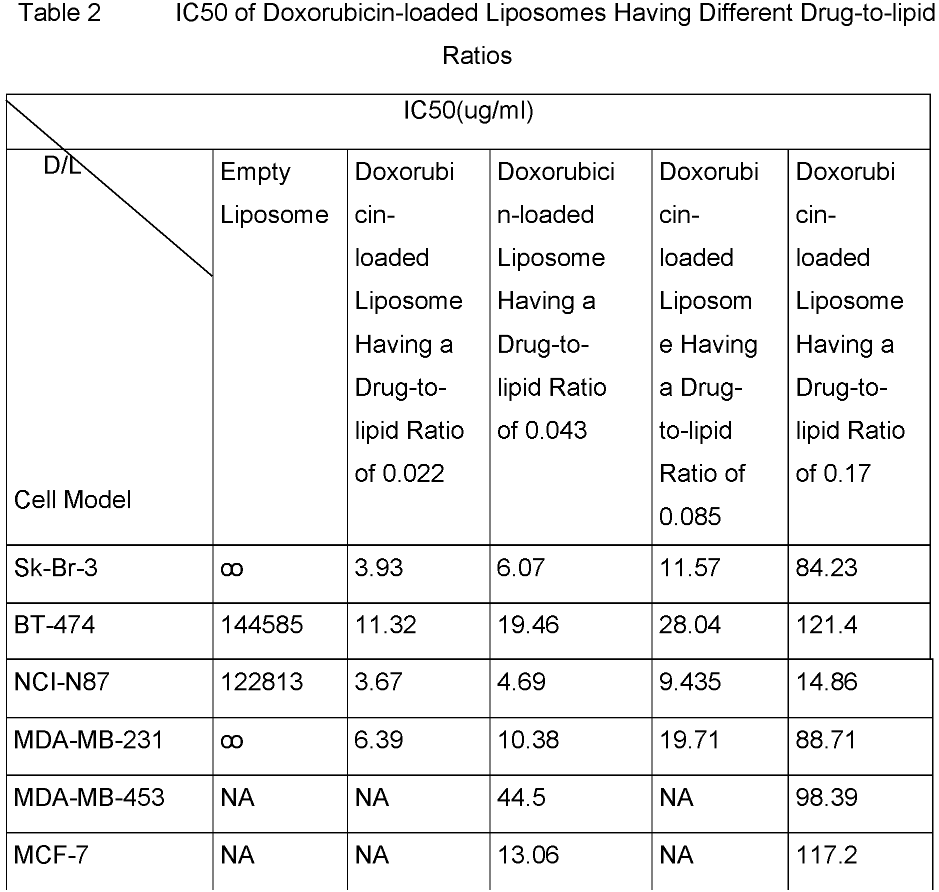

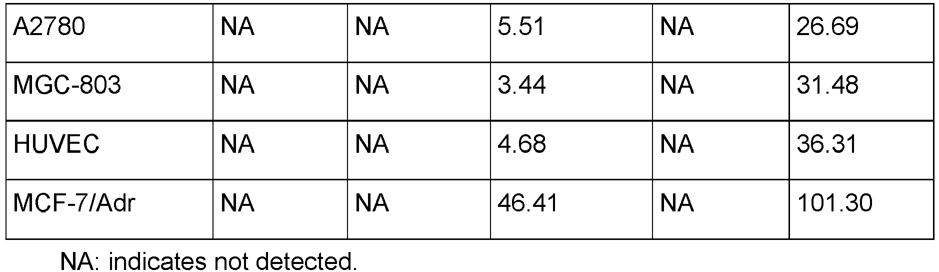

In addition, in order to more fully validate the cytotoxic effect of doxorubicin-loaded liposomes having different drug-to-lipid ratios on different cell lines, the inventors of the present disclosure set up more experimental groups with different drug-to-lipid ratios, and tested their IC50 values on different cell lines.

The test results are as shown in Table 3 The results showed that liposomes having lower drug-to-lipid ratios had better cytotoxic effects, especially liposomes having drug-to-lipid ratios below 0.1.

**Table 3 IC50 of Doxorubicin-loaded Liposomes Having Different Drug-to-lipid Ratios**

| IC50 | | | | | | | | |
|---|---|---|---|---|---|---|---|---|
| **Drug-to-lipid Ratio** | 0.01 | 0.022 | 0.043 | 0.085 | 0.1 | 0.12 | 0.15 | 0.17 |
| Sk-Br-3 | 19.77 | 3.93 | 6.07 | 11.57 | 15.54 | 24.13 | 26.91 | 84.23 |
| BT-474 | 36.5 | 11.32 | 19.46 | 28.04 | 135.1 | 140.8 | 136.0 | 121.4 |
| NCI-N87 | 5.805 | 3.67 | 4.69 | 9.435 | 11.62 | 15.00 | 11.98 | 14.86 |
| MDA-M B-231 | 42.75 | 6.39 | 10.38 | 19.71 | 142.1 | 156.9 | 127.8 | 88.71 |

### Example 3 Preparation of a HER2/neu Antibody-targeted Doxorubicin-loaded Liposome and Efficacy Performance in Cells 3.1 Preparation of HER2/neu antibody-targeted doxorubicin-loaded liposomes

A HER2/neu antibody-targeted doxorubicin-loaded liposome was prepared, and the lipid composition was HSPC, cholesterol (Chol), 1,2-distearoyl-rac-glycero-3-PEN-polyethyleneglycol-2000 (DSPE-MPEG2000), and a HER/neu antibody fragment-conjugated lipid molecule, wherein the range of molar ratio of each component was (30 -80):(5 - 40):(0.1 - 10):(0.01 - 0.1), wherein the HER2/neu antibody fragment-conjugated lipid molecule was 1,2-distearoyl-sn-glycero-3-phosphoethanolamine-N-[maleimide(polyethylene glycol)-2000]-HER2/neu antibody fragment (HER2-DSPE-PEG2000). The HER2/neu antibody fragment was reacted with lipid molecule DSPE-PEG2000-mal containing maleimide group through free sulfhydryl groups to form a thioether bond. Then, HER2-DSPE-PEG2000 was co-incubated with the doxorubicin-loaded liposome having a drug-to-lipid ratio of 0.043 prepared in Example 1 to obtain a specific HER2/neu antibody-targeted drug-loaded liposome.

### 3.2 Performance of HER2/neu Antibody-targeted Doxorubicin-loaded Liposomes in Tumor Cells

In the present example, the cytotoxic effect of the HER2/neu antibody-targeted doxorubicin-loaded liposome prepared in Example 3.1 on tumor cells having different levels of HER2/neu expression was assessed.

Tumor cell lines: Tumor cell lines included SK-Br-3, NCI-N87, BT474, MDA-MB-453, MCF-7, MGC-803, A2780, MDA-MB-231, and a normal cell line, HUVEC, with specific cell HER2/neu expression levels shown in Table 4.

**Table 4 HER2/neu Expression Levels on Different Cell Surfaces**

| **Cell Line** | **HER2/neu Expression (rMFI, FCS)** | **Score** |
|---|---|---|
| SK-Br-3 | 62.55 | 3+ |
| NCI-N87 | 61.85 | 3+ |
| BT474 | 51.15 | 3+ |
| MDA-MB-453 | 17.71 | 2+ |
| MCF-7 | 2.59 | 1+ |
| A2780 | 4.48 | 1+ |
| MGC-803 | 2.84 | 1+ |
| HUVEC | 4.11 | 1+ |
| MDA-MB-231 | 1.33 | 0 |

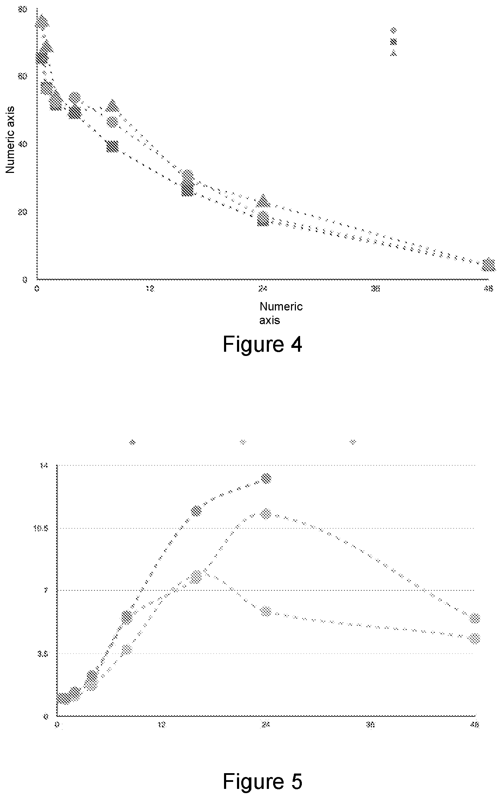

The cytotoxic effect of the HER2/neu antibody-targeted doxorubicin-loaded liposome on tumor cells was assessed using the CellTiter-Glo^{®} Luminescent Cell Viability Assay kit from Promega Corporation. Trastuzumab monoclonal antibody (trastuzumab) was set as a control. At the same time, non-targeted doxorubicin-loaded liposomes having drug-to-lipid ratios of 0.043 and 0.17 were compared. Graph Prism 8.0 analysis software was used to fit cell cytotoxicity curves and calculate the respective IC50 values. See Figure 5 for the results.

The results showed that HER2/neu antibody-targeted doxorubicin-loaded liposome having a lower drug-to-lipid ratio had better cytotoxic effects on tumor cells, and targeted HER2/neu-positive tumors. The cytotoxic activity of the HER2/neu antibody-targeted doxorubicin-loaded liposome in HER2/neu-overexpressing tumor cells was 3 - 10-fold higher than that of the doxorubicin-loaded liposome having a drug-to-lipid ratio of 0.043, and was more than 10-fold higher than that of the doxorubicin-loaded liposome having a drug-to-lipid ratio of 0.17.

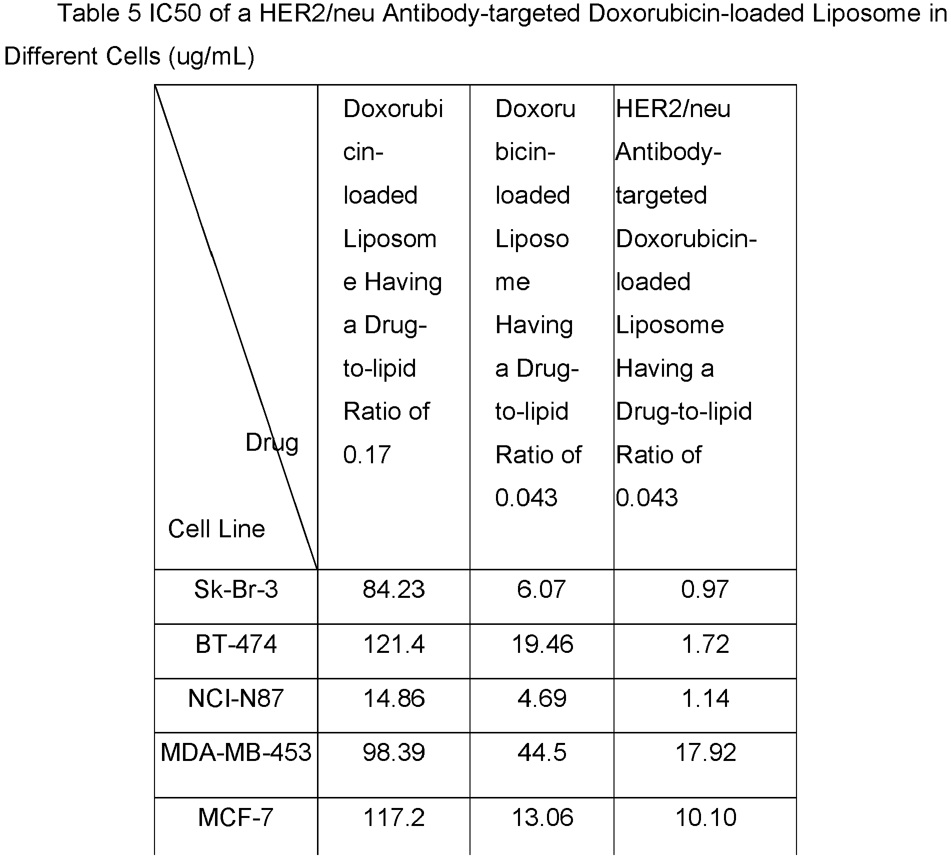

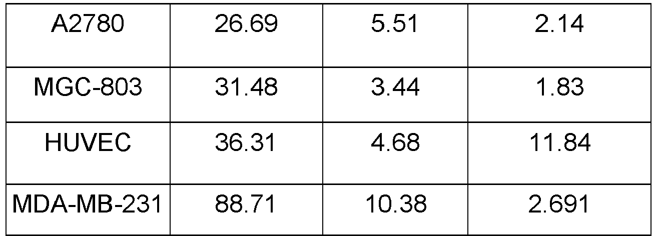

### 3.3 Performance of a HER2/neu Antibody-targeted Doxorubicin-loaded Liposome in Adriamycin-resistant Tumor Cell Lines

Refer to 3.2 for the specific implementation procedures. In order to investigate the performance of the HER2/neu antibody-targeted doxorubicin-loaded liposome in Adriamycin-resistant tumor cell lines, MCF-7/Adr Adriamycin-resistant breast cancer lines were used for testing. The cytotoxicity results showed that the IC50 values of the HER2/neu antibody-targeted doxorubicin-loaded liposome, doxorubicin-loaded liposomes having drug-to-lipid ratios of 0.043 and 0.17, as well as trastuzumab monoclonal antibody (trastuzumab) in MCF-7/Adr were 13.70 µg/mL, 46.41 µg/mL, and 101.30 µg/mL, respectively (as shown in Figure 3), indicating that the HER2/neu antibody-targeted doxorubicin-loaded liposome still had strong cytotoxic effects on Adriamycin-resistant cell lines. Therefore, it has potential clinical efficacy in the treatment of patients with Adriamycin-resistant tumors.

### Example 4 Pharmacokinetic Performance of Doxorubicin-loaded Liposomes Having Different Drug-to-lipid Ratios

The in vivo pharmacokinetic profile of doxorubicin-loaded liposomes having different drug-to-lipid ratios was studied in tumor-bearing mouse models, wherein trace amounts of deuterated cholesterol molecular markers were added to the liposomes to detect changes in exogenous lipid concentrations.

Construction of BT474 breast cancer xenograft model in Balb/C-Nude mice: Female SPF grade Balb/C-Nude mice aged 4 - 5 weeks, weighing about 18 g were purchased and adaptively fed in the animal feeding room for about one week. BT474 cells were grown to the exponential growth phase, and the cells were digested with trypsin. The cells were centrifuged, collected, and washed with PBS buffer for two - three times. The cells were resuspended in PBS buffer and counted, and the cell density was adjusted to 1 * 10^8 cells/mL. The matrigel was removed from the -20°C refrigerator in advance and thawed on ice, and the cell suspension was mixed with the matrigel in an equal volume of 1:1. The corresponding volume of cell suspension was pipetted into a 1 mL syringe based on a cell volume of 5 * 10⁶ - 1 * 10⁷, and the cell suspension was subcutaneously inoculated into the right axilla of the nude mice. After inoculation, the injection site was compressed for 30 s to avoid reflux of the cell solution. After inoculation, a significant subcutaneous papule was observed. The inoculated mice were observed regularly and tumor growth was monitored.

Dosing: The first group was given a doxorubicin-loaded liposome having a drug-to-lipid ratio (high drug-to-lipid ratio) of 0.17 based on the mice's weight via tail intravenous injection, and the drug dose was 5 mg/kg; the second group was given a doxorubicin-loaded liposome having a drug-to-lipid ratio (medium drug-to-lipid ratio) of 0.085 based on the mice's weight via tail intravenous injection, and the drug dose was 5 mg/kg; the third group was given a doxorubicin-loaded liposome having a drug-to-lipid ratio of 0.043 (low drug-to-lipid ratio) based on the mice's weight via tail intravenous injection, and the drug dose was 5 mg/kg.

Blood collection: Blood was collected from four different groups that received doxorubicin-loaded liposomes having different drug-to-lipid ratios at 0.5, 1, 2, 4, 6, 8, 12, 24, 48 and 72 hours after dosing, collected in EDTA anticoagulant tubes, placed on ice, and centrifuged at 4°C and 3,000 rpm for 30 min. The upper layer of plasma was carefully pipetted into a new labeled centrifuge tube, and subsequently tested or stored in a -80°C refrigerator.

Pretreatment of plasma sample: 40 µL of plasma sample was accurately measured into a centrifuge tube; 160 µL of acetonitrile-methanol (1:1, v/v) was added according to the ratio of plasma to organic reagent 1:4, vortexed for 1 - 2 min to fully precipitate protein in plasma, and centrifuged at a rotational speed of 1,000 × g at 4°C for 10 min. The supernatant was carefully pipetted into a new centrifuge tube, while paying attention to avoid pipetting the protein layer in this step, and centrifuged at a rotational speed of 1,000 × g at 4°C for 10 min. The solution was filtered using 0.22 µm organic filter head, and 50 uL was placed in a sample vial, and loaded for testing.

Testing: High performance liquid chromatography conditions (refer to Chinese Pharmacopoeia): Athena C18 column (120Å, 4.6×150 mm, 5 µm) was used and octadecyl silane chemically bonded to porous silica was used as the filler; sodium dodecyl sulfate solution (1.44 g of sodium dodecyl sulfate and 0.68 mL of phosphoric acid were taken and 500 mL of water was added to dissolve)-acetonitrile-methanol (500:500:60) was used as the mobile phase; the detection wavelength was 254 nm; the detection flow rate was 1.0 mL/min; the loading volume was 10 µL.

Analysis: The plasma concentration results of liposomes having different drug-to-lipid ratios in rats are shown in Figure 4, and the changes in the ratio of drug concentration to deuterated cholesterol concentration in blood samples are shown in Figure 5. Changes in the ratio of drug concentration to deuterated cholesterol concentration in blood were used to indicate the process of drug release from liposomes. Liposomes having different drug-to-lipid ratios were observed to have different in vivo release processes. Liposomes having low drug-to-lipid ratios had slower release. This provides better sustained release.

### Example 5 In Vivo Pharmacodynamic Experiment of Doxorubicin-loaded Liposomes Having Different Drug-to-lipid Ratios

### 5.1.Xenograft model construction

Construction of BT474 breast cancer xenograft model in Balb/C-Nude mice: Female SPF grade Balb/C-Nude mice aged 4 - 5 weeks, weighing about 18 g were purchased and adaptively fed in the animal feeding room for about one week. BT474 cells were grown to the exponential growth phase, and the cells were digested with trypsin. The cells were centrifuged, collected, and washed with PBS buffer for two - three times. The cells were resuspended in PBS buffer and counted, and the cell density was adjusted to 1 * 10^8 cells/mL. The matrigel was removed from the -20°C refrigerator in advance and thawed on ice, and the cell suspension was mixed with the matrigel in an equal volume of 1:1. The corresponding volume of cell suspension was pipetted into a 1 mL syringe based on a cell volume of 5 * 10⁶ - 1 * 10⁷, and the cell suspension was subcutaneously inoculated into the right axilla of the nude mice. After inoculation, the injection site was compressed for 30 s to avoid reflux of the cell solution. After inoculation, a significant subcutaneous papule was observed. The inoculated mice were observed regularly and tumor growth was monitored.

### 5.2 Dosing and efficacy evaluation:

When the tumor size reached 100 - 200 mm³, the mice were randomly divided into three groups, with at least six mice in each group, and the three groups were the control group, the 0.17 drug-to-lipid ratio doxorubicin-loaded liposome group, the 0.043 drug-to-lipid ratio doxorubicin-loaded liposome group, and the HER2/neu antibody-targeted 0.043 drug-to-lipid ratio doxorubicin liposome group.

After grouping, the control group was given empty liposomes via tail intravenous injection. The 0.17 drug-to-lipid ratio doxorubicin-loaded liposome group was given a doxorubicin-loaded liposome having a drug-to-lipid ratio of 0.17 at a dose of 2 mg/kg based on the body weight of nude mice via tail intravenous injection; the 0.043 drug-to-lipid ratio doxorubicin-loaded liposome group was also given a doxorubicin-loaded liposome having a drug-to-lipid ratio of 0.043 at a dose of 2 mg/kg based on the body weight of nude mice via tail intravenous injection; the HER2/neu antibody-targeted 0.043 drug-to-lipid ratio doxorubicin-loaded liposome group was also given a HER2/neu antibody-targeted doxorubicin-loaded liposome having a drug-to-lipid ratio of 0.043 at a dose of 2 mg/kg based on the body weight of nude mice via tail intravenous injection The drug was dosed once a week, for a total of five times. The body weight and tumor growth curve of mice were recorded every other day, and the mice were euthanized after the end of dosing.

The inhibitory effect of the test article on the breast cancer BT474 xenograft model in mice was mainly tested.

Tumor volume and weight measurement of tumor-bearing mice: Measured using a vernier caliper every other day, and tumor volume was calculated using the formula V = 0.5a × b², where a and b represent the long diameter and width of the tumor, respectively.

Tumor growth inhibition rate TGI(%) = [1 - (Dᵢ - D₀) / (Cᵢ - C₀)] × 100, wherein Dᵢ-D₀ > 0. D₀ is the mean tumor volume at the first dose of the dosing group (Drug), Dᵢ is the mean tumor volume at a certain measurement after the start of dosing in the dosing group; C₀ is the mean tumor volume at the first dose of the control group (Control), and Cᵢ is the mean tumor volume at a certain measurement after the start of dosing in the control group.

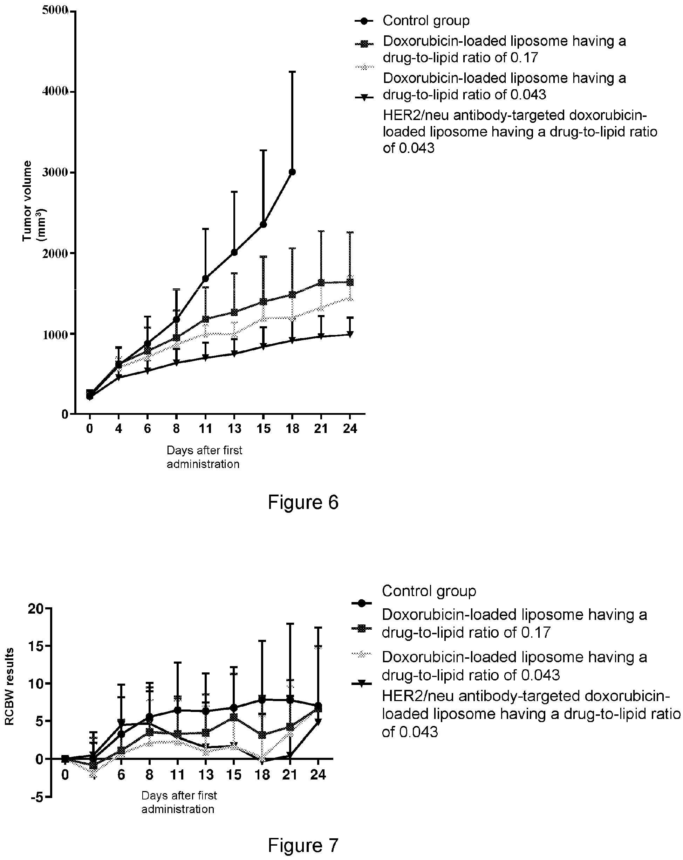

The in vivo pharmacodynamics results on the BT474 breast cancer xenograft model in Balb/C-Nude mice are shown in Figure 6 and Figure 7. As shown in Figure 6, the tumor growth inhibition rates (TGI%) of doxorubicin-loaded liposome having a drug-to-lipid ratio of 0.17, doxorubicin-loaded liposome having a drug-to-lipid ratio of 0.043, and HER2/neu antibody-targeted doxorubicin-loaded liposome having a drug-to-lipid ratio of 0.043 on Day 18 after dosing were 50.55%, 60.12%, and 69.66%, respectively. The results showed that doxorubicin-loaded liposome having a drug-to-lipid ratio of 0.043 had stronger tumor growth inhibition, and HER2/neu antibody-targeted doxorubicin-loaded liposome having a drug-to-lipid ratio of 0.043 had even better tumor growth inhibition. As shown in Figure 7, Balb/C-Nude mice experienced weight loss after dosing, but did not demonstrate significant toxicity (weight loss < 10%), and the weight of mice returned to normal at the end of dosing.

### Example 6: In Vivo Animal Pharmacodynamic Experiment of a HER2/neu Antibody-targeted Doxorubicin-loaded Liposome Having a Low Drug-to-lipid Ratio

### 6.1. Construction of NCI-N87 breast cancer xenograft model in Balb/C-Nude mice

A xenograft tumor model was constructed in Balb/C-Nude mice using HER2-overexpressing NCI-N87 gastric cancer cell lines on the cell surface.

Female SPF grade Balb/C-Nude mice aged 4 - 5 weeks, weighing about 18 g were purchased and adaptively fed in the animal feeding room for about one week. NCI-N87 cells were grown to the exponential growth phase, and the cells were digested with trypsin. The cells were centrifuged, collected, and washed with PBS buffer for two - three times. The cells were resuspended in PBS buffer and counted, and the cell density was adjusted to 2 * 10^8 cells/mL. The matrigel was removed from the -20°C refrigerator in advance and thawed on ice, and the cell suspension was mixed with the matrigel in an equal volume of 1:1. The corresponding volume of cell suspension was pipetted into a 1 mL syringe based on a cell volume of 1 * 10^7, and the cell suspension was subcutaneously inoculated into the right axilla of the nude mice. After inoculation, the injection site was compressed for 30 s to avoid reflux of the cell solution. After inoculation, a significant subcutaneous papule was observed. The inoculated mice were observed regularly and tumor growth was monitored.

### 6.2. Dosing and efficacy evaluation

When the tumor size reached 100 - 200 mm³, the mice were randomly divided into three groups, with at least six mice in each group, and the three groups were the control group, the 0.17 drug-to-lipid ratio doxorubicin-loaded liposome group, and the HER2/neu antibody-targeted 0.043 drug-to-lipid ratio doxorubicin liposome group.

After grouping, the control group was given empty liposomes via tail intravenous injection. The 0.17 drug-to-lipid ratio doxorubicin-loaded liposome group was given a doxorubicin-loaded liposome having a drug-to-lipid ratio of 0.17 at a dose of 2 mg/kg based on the body weight of nude mice via tail intravenous injection; the HER2/neu antibody-targeted 0.043 drug-to-lipid ratio doxorubicin-loaded liposome group was also given a HER2/neu antibody-targeted doxorubicin-loaded liposome having a drug-to-lipid ratio of 0.043 at a dose of 0.5, 2, 5, or 10 mg/kg (mpk) based on the body weight of nude mice via tail intravenous injection. At the same time, trastuzumab monoclonal antibody was set as the control, and 5 mg/kg was given, once a week (qw), for a total of five times. The body weight and tumor growth curve of the mice were recorded every other day, and the mice were euthanized after the end of dosing.

The inhibitory effect and complete curative ability of the test article on the NCI-N87 gastric cancer xenograft model in mice were mainly tested.
(1) Tumor volume measurement: Measured using a vernier caliper every other day, and tumor volume was calculated using the formula V = 0.5a × b², where a and b represent the long diameter and width of the tumor, respectively.
(2) Tumor growth inhibition rate TGI(%) = [1 - (Dᵢ - D₀) / (Cᵢ - C₀)] × 100, wherein Dᵢ - Do > 0. Do is the mean tumor volume at the first dose of the dosing group (Drug), Dᵢ is the mean tumor volume at a certain measurement after the start of dosing in the dosing group; C₀ is the mean tumor volume at the first dose of the control group (Control), and Cᵢ is the mean tumor volume at a certain measurement after the start of dosing in the control group.
(3) The body weight of all tumor-bearing mice was measured every other day. At the same time, the rate of change in body weight in mice after dosing was also calculated: RCBW(%) = (BWᵢ - BW₀) / BW₀ × 100, BWᵢ is the mean body weight at a certain measurement after the start of dosing, and BWo is the mean body weight at the first dose.

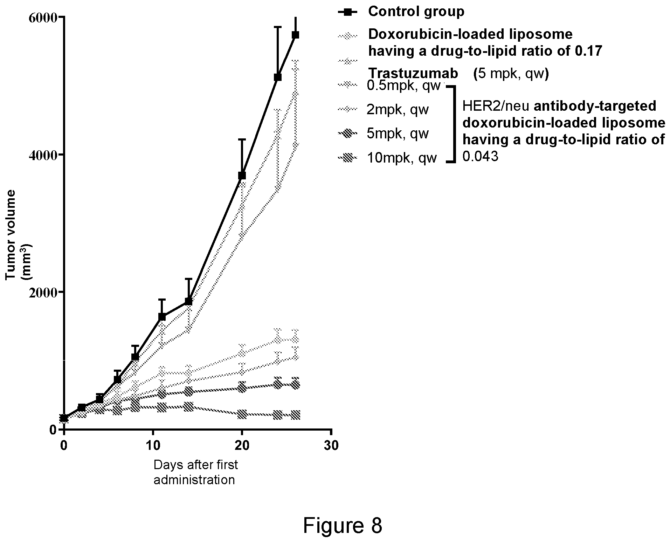

The in vivo pharmacodynamics results on the NCI-N87 gastric cancer xenograft model in Balb/C-Nude mice are shown in Figure 8. The tumor growth curve (Figure 8) showed that the antibody-targeted doxorubicin-loaded liposome having a low drug-to-lipid ratio (0.043) had better in vivo tumor growth inhibition on the HER2/neu-overexpressing NCI-N87 gastric cancer xenograft model, and had a dose-dependent relationship.

### Example 7 Preparation of Irinotecan-loaded Liposomes Having Different Drug-to-lipid Ratios

HSPC, Chol and DSPE-MPEG2000 were precisely weighed, added into a glass flat-bottomed flask, an appropriate amount of anhydrous ethanol was added, and heated in a water bath to fully dissolve the lipids. 30 mL of 500 mM 1, 3-propane disulfonic acid triethylamine was added as the hydration medium, and stirred for hydration at 65°C for 45 minutes. Homogeneous small unilamellar vesicles were formed by sequential extrusion through 100 nm and 80 nm polycarbonate filter membranes.

10 mM, pH 7.0 HEPES buffer was used as the dialysis medium to form the propane disulfonic acid triethylamine gradient of the internal and external aqueous phase of the liposome. An irinotecan solution with a concentration of 10 mg/mL was prepared, and the drug was loaded according to the drug-to-lipid ratios of 0.01, 0.02, 0.04, 0.075, 0.15 and 0.3, and heated and incubated in a 60°C water bath for 45 min. Then, 10 mM, pH 7.0 HEPES buffer was used as the dialysis medium to remove the free drug in the external aqueous phase to obtain irinotecan-loaded liposomes having different specific drug-to-lipid ratios of 0.01, 0.02, 0.04, 0.075, 0.15 and 0.3.

The physical parameters of irinotecan-loaded liposomes having drug-to-lipid ratios of 0.075, 0.15 and 0.3 are shown in Table 6. The particle sizes were similar, the PDI distribution was all less than 0.1, and the encapsulation rates were all greater than 95%.

**Table 6 Physical Parameters of Irinotecan-loaded Liposomes Having Different Drug-to-lipid Ratios**

| Drug-to-lipid Ratio | Average Particle Size (nm) | PDI | Encapsulation Rate (%) |
|---|---|---|---|
| 0.075 | 82 | 0.07 | 98.5 |
| 0.15 | 82 | 0.07 | 99.5 |
| 0.3 | 83 | 0.069 | 98.9 |

The cytotoxicity of irinotecan-loaded liposomes having different drug-to-lipid ratios was tested on different cell models using the method according to Example 2, and the results obtained are shown in Table 7. The results showed that the irinotecan-loaded liposome having a drug-to-lipid ratio of 0.15 had the best cytotoxicity.

**Table 7 IC50 of Irinotecan-loaded Liposomes Having Different Drug-to-lipid Ratios on NCI-N87 Cell Model**

| Cytotoxic Effects of IRI-Lip Having Different Drug-to-lipid Ratios on NCI-N87 Cells | |
|---|---|
| D/L ratio | IC50 (µM) |
| 0.3 | 180.3 |
| 0.15 | 67.06 |
| 0.075 | 131.0 |
| 0.04 | 126.2 |
| 0.02 | 156.5 |
| 0.01 | 153.9 |

### Example 8: Cytotoxicity Experiment of Antibody-targeted Irinotecan-loaded Liposomes Having Different Drug-to-lipid Ratios

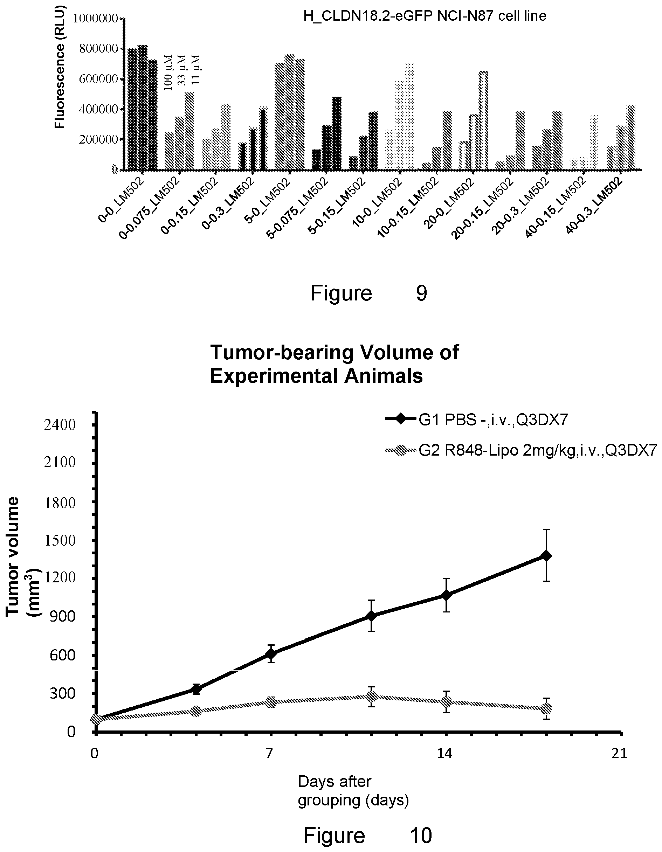

The present example used an NCI-N87 tumor cell line purchased from the cell bank of the Chinese Academy of Sciences. Antibody-targeted irinotecan-loaded liposomes having different drug-to-lipid ratios were prepared and assessed for cytotoxic effect on NCI-N87. The test method was consistent with that in Example 2, i.e., the CellTiter-Glo^{®} Luminescent Cell Viability method was used. The results are as shown in Figure 9, the antibody-targeted irinotecan-loaded liposome having a drug-to-lipid ratio of 0.15 had better cytotoxicity on NCI-N87 cells.

### Example 9 Preparation of a R848-loaded Liposome Having a Low Drug-to-lipid Ratio and In Vivo Animal Pharmacodynamic Experiment

### 9.1 Preparation of a R848-loaded liposome having a low drug-to-lipid ratio

285.7 mg of HSPC, 95 mg of Chol and 19 mg of DSPE-MPEG2000 were weighed, 1 mL of anhydrous ethanol was added, and heated in a water bath to 60°C. Then, a magnetic stirring bar was added for stirring until complete dissolution, resulting in a clear liquid mixture. At the same time, 6.607 g of ammonium sulfate was placed in a glass bottle, added with 100 mL of ultrapure water, and stirred until dissolution, and the pH was then adjusted to 5.0 to obtain an ammonium sulfate solution with a concentration of 500 mM; this solution was prepared just before use. The dissolved lipid mixture was injected into 9 mL of the ammonium sulfate aqueous solution using a syringe, then stirred to hydrate at 60°C for 45 min, extruded six times using 100 nm and 80 nm polycarbonate filter membranes, and dialysis was then performed using a 10 kD dialysis membrane in a HEPES buffer (10 mM, 0.9% NaCl, pH 5.5) to obtain an empty liposome.

In addition, 6.25 mL of 1 mg/mL R848 solution was taken and added to 3.75 mL of empty liposome with a lipid concentration of 40 mg/mL to obtain a total volume of 10 mL. After stirring and loading the drug at room temperature for 30 min, the free drug was removed using a 100kD dialysis bag to prepare an R848-loaded liposome having a drug-to-lipid ratio of 0.09 (R848-Lipo). The particle size was 74.89 nm, and the PDI was less than 0.026.

### 9.2 Establishing a B-hHER2 MC38 colon cancer model using B-hCD3E mice and testing the in vivo pharmacodynamics of the drug

B-hHER2 MC38 cells resuspended in PBS were subcutaneously inoculated at 1x 10⁶/0.1 mL/mouse into the right side of the back of B-hCD3E mice. When the mean tumor volume reached about 80 -150 mm³, suitable mice were selected for enrollment according to the tumor volume and mouse weight, and randomly assigned to two experimental groups, namely the control group and the experimental group, with six mice in each group. After grouping, the control group was given PBS via tail intravenous injection, and the experimental group was given the R848-loaded liposome having a drug-to-lipid ratio of 0.09 at a dose of 2 mg/kg based on the body weight of the mice via tail intravenous injection, once every three days, for a total of seven times.

After grouping, the tumor volume was measured using a vernier caliper twice a week, and the long and short diameters of the tumor were measured. The volume calculation formula was Tumor volume = 0.5 × long diameter × short diameter².

The in vivo pharmacodynamic results of the B-hHER2 MC38 colon cancer model in B-hCD3E mice are shown in Figure 10. The tumor growth curve showed that the R848 liposome has a significant inhibitory effect on the growth of B-hHER2 MC38 colon cancer in vivo.

### Example 10 Preparation and Testing of Cisplatin-loaded Liposomes Having Different Drug-to-lipid Ratios

HSPC, Chol and DSPE-MPEG2000 were precisely weighed, added into a glass flat-bottomed flask, an appropriate amount of anhydrous ethanol was added, and heated in a water bath to fully dissolve the lipids. The solution was injected into a 50°C 1 mg/mL, 2 mg/mL, and 5 mg/mL cisplatin solution at a rate of 30 mL/min, the mixed solution was extruded using 100 nm and 80 nm polycarbonate filter membranes, and the unencapsulated drug was removed by dialysis to obtain cisplatin-loaded liposomes having a particle size of about 80 nm and drug-to-lipid ratios of 0.01, 0.05, and 0.08.

All literature mentioned in the present disclosure is cited as a reference in the present application, as if each piece of literature is cited separately as a reference. It should also be understood that, after reading the above description of the contents of the present disclosure, those skilled in the art may make various changes or modifications to the present disclosure, and such equivalents similarly fall within the same scope defined by the claims appended to the present application.

## Claims

1. A pharmaceutical composition, **characterized in that** it comprises a1) an anti-tumor drug; a2) a liposome as a carrier; wherein the molar ratio (drug-to-lipid ratio) of the anti-tumor drug to the lipid carrier is between 0.01 and 0.15.

2. The pharmaceutical composition according to Claim 1, **characterized in that** the range of the drug-to-lipid ratio is 0.02 - 0.1, preferably 0.022 - 0.085.

3. The pharmaceutical composition according to Claim 1, **characterized in that** the anti-tumor drug is selected from the following group: anthracyclines, platinum-based drugs, fluorouracils, camptothecins, taxanes, or combinations thereof.

4. The pharmaceutical composition according to Claim 1, **characterized in that** the anti-tumor drug further includes immunomodulatory drugs.

5. The pharmaceutical composition according to Claim 1, **characterized in that** the anti-tumor drug is selected from the following group: doxorubicin, irinotecan, cisplatin, paclitaxel, resiquimod, or combinations thereof.

6. The pharmaceutical composition according to Claim 1, wherein the pharmaceutical composition has one or more characteristics selected from the following group:
(1) the drug in the pharmaceutical composition having an encapsulation rate > 90%;
(2) the pharmaceutical composition having a particle size between 60 and 180 nm, preferably between 80 and 120 nm;
(3) the pharmaceutical composition having a PDI between 0.01 and 0.2, and more preferably between 0.01 and 0.15.

7. The pharmaceutical composition according to Claim 1, **characterized in that** the lipid carrier is selected from the following group of ingredients consisting of: phosphatidylcholine, phosphatidylglycerol, phosphatidylserine, phosphatidylethanolamine, sphingomyelin, cholesterol, polyethylene glycol glycerol fatty acid ester, and polyethylene glycol glycerol phosphatidylethanolamine.

8. The pharmaceutical composition according to Claim 1, **characterized in that** the lipid carrier includes phosphatidylcholine and cholesterol, wherein the phosphatidylcholine is selected from the following group: hydrogenated soybean phosphatidylcholine (HSPC), soybean phosphatidylcholine (SPC), hydrogenated egg phosphatidylcholine, dilauryl phosphatidylcholine, dimyristoyl phosphatidylcholine, dipalmitoyl phosphatidylcholine, distearoyl phosphatidylcholine, 1-myristoyl-2-palm itoyl-sn-glycero-3-phosphocholine, 1-palmitoyl-2-stearoyl-sn-glycero-3-phosphocholine, 1-palmitoyl-2-oleoyl-sn-glycero-3-phosphocholine, 1-stearoyl-2-linoleoyl-sn-glycero-3-phosphocholine, dioleoyl phosphatidylcholine, or combinations thereof.

9. The pharmaceutical composition according to Claim 1, **characterized in that** the method for preparation of the lipid carrier includes the following steps:
(a) mixing the liposomal raw material and cross-linking agent with ethanol to obtain the lipid raw material alcohol phase solution;
(b) providing an acidic aqueous phase solution having a pH of 3.5 - 6.0;
(c) injecting a lipid raw material alcohol phase solution into an acidic aqueous phase solution, stirring and extruding to obtain the liposome as a carrier.

10. The pharmaceutical composition according to Claim 1, **characterized in that** the lipid carrier has a conjugated targeting group thereon.

11. The pharmaceutical composition according to Claim 10, **characterized in that** the targeting group is selected from the following group: polypeptides, proteins, antibody fragments, preferably HER2/neu antibody fragments.

12. A method for preparing the pharmaceutical composition according to Claim 1, **characterized in that** including the steps:
(1) mixing and incubating an anti-tumor drug solution with a lipid carrier dispersion to obtain the pharmaceutical composition; or
(2) adding an anti-tumor drug to the lipid raw material alcohol phase solution or aqueous phase solution to obtain the pharmaceutical composition in one step during the preparation of a lipid carrier.

13. The method for preparation according to Claim 11, **characterized in that** the method further includes the steps: mixing and incubating the obtained drug-loaded liposome with a modified targeting group to obtain the targeted pharmaceutical composition.

14. A use for the pharmaceutical composition according to Claim 1, **characterized in that** the use is for preparing b1) an inhibitor that inhibits tumor cell proliferation; b2) an anti-tumor drug.

15. The use according to Claim 14, **characterized in that** the dosage form of the pharmaceutical composition is non-oral.

16. The use according to Claim 14, **characterized in that** the tumor is selected from the group: breast cancer, gastric cancer, ovarian cancer, liver cancer, melanoma, colorectal cancer, or combinations thereof.

17. A method for inhibiting tumor cell growth in vitro, **characterized in that** including the steps: administering to the cell a medically effective amount of the pharmaceutical composition according to Claim 1.

18. A method for preventing and/or treating a tumor, **characterized in that** including the steps of: administering a medically effective amount of the pharmaceutical composition according to Claim 1 to a subject in need thereof.
